# EUROPÄISCHE PATENTSCHRIFT

(11) **EP 2 675 496 B1**
(45) Veröffentlichungstag und Bekanntmachung des Hinweises auf die Patenterteilung: **27.06.2018**
(21) Anmeldenummer: 12706458.2
(22) Anmeldetag: 17.02.2012
(51) Int. Cl.: A61M 1/14, G06F 19/00

(54) **MEDIZINISCHES GERÄT MIT TOUCHSCREEN UND VERFAHREN**
MEDICAL DEVICE WITH TOUCHSCREEN, AND METHOD
APPAREIL MÉDICAL DOTÉ D'UN ÉCRAN TACTILE ET PROCÉDÉ CORRESPONDANT

(30) Priorität: 18.02.2011 DE 102011011769; 18.02.2011 US 201161444316 P
(43) Veröffentlichungstag der Anmeldung: 25.12.2013
(73) Patentinhaber: Fresenius Medical Care Deutschland GmbH, 61352 Bad Homburg (DE)
(72) Erfinder: BLUEMLER, Holger, 61184 Karben (DE); WEHMEYER, Wolfgang, 72076 Tübingen (DE)
(74) Vertreter: Lindemann, Robert
(86) Internationale Anmeldenummer: PCT/EP2012/000708
(87) Internationale Veröffentlichungsnummer: WO 2012/110250

(56) Entgegenhaltungen:
- WO-A2-2008/030594
- DE-A1-102008 058 568
- US-A1- 2009 294 339
- "Touch-Screen mit Piezo-Aktor zur taktilen RÃ 1/4 ckmeldung", IP.COM JOURNAL, IP.COM INC., WEST HENRIETTA, NY, US, 17. Februar 2011 (2011-02-17), XP013142530, ISSN: 1533-0001

## Beschreibung

Die Erfindung betrifft ein medizintechnisches Gerät, insbesondere ein Blutbehandlungsgerät mit mindestens einem Touchscreen und ein Verfahren zur Anzeige und Eingabe von Informationen bei einem Blutbehandlungsgerät mit mindestens einem Touchscreen.

Medizintechnische Geräte mit berührempfindlichen Displays (Touchscreen) als gleichzeitiges Mittel zur Anzeige von Informationen und zur Aufnahme von Bedienereingaben sind im Stand der Technik weit verbreitet. Ein solches medizintechnischen Gerätes ist beispielsweise die Hämodialysemaschine 5008 von Fresenius Medical Care.

Medizintechnische Geräte mit Touchscreens bieten dem Bediener eine komfortable, variable und durch die ebene und undurchbrochene Oberfläche eine hygienische Bedieneroberfläche.

Touchscreens für medizintechnische Geräte bieten im Wesentlichen bislang nur glatte und ebene Oberflächen. Der Bediener erkennt durch optische Anzeige eines Schaltfelds den Bereich des Displays, auf dem ein Fingerdruck erwartet wird. Eine taktile Rückmeldung, ob z.B. dieser erwartete Bereich auch getroffen wurde, erfolgt nicht. Eine solche taktile Rückmeldung bieten konventionelle mechanische Schalter, bei denen eine Betätigung einen fühlbaren Kraftaufwand erfordert und/oder durch den Schaltweg fühlbar ist. Nachteilig bei solchen mechanischen Schaltern ist deren häufige bauartbedingte Eigenschaft, die Oberfläche des medizintechnischen Gerätes zu durchbrechen, was eine hygienische Säuberung der Oberfläche erschwert. Darüber hinaus bieten mechanische Schalter nicht die Variabilität einer softwaregesteuerten Schaltmatrix eines Touchscreens, bei dem Anzahl, Größe und Position der virtuellen Schalter auf dem Touchscreen flexibel verändert werden können.

Es ist für die Sicherheit eines medizintechnischen Geräts wesentlich, dass die Eingabe und die Anzeige von Informationen auf einem Touchscreendisplay sicher und eindeutig funktioniert

Hierfür ist es sinnvoll, die Eingabemöglichkeit über den Touchscreen redundant zu gestalten, um bei Ausfall der Eingabefunktionalität des Touchscreens wichtige Eingaben, beispielsweise zur gefahrlosen Beendung der Behandlung eines Patienten, eine weitere Eingabemöglichkeit zu haben. Dies wird im Stand der Technik beispielsweise durch zusätzliche mechanische Schalter oder Taster ermöglicht. Nachteilig erweisen sich hierbei die schon weiter oben erwähnten Eigenschaften mechanischer Schalter

Aus dem Bereich der Telekommunikation und der Computertechnik sind Geräte bekannt, die über einen Touchscreen mit taktilem Feedback verfügen. Die WO2009/085060 beschreibt ein solches Gerät

Die taktile Rückmeldung erfolgt bei solchen Geräten, um dem Benutzer beim Fingerdruck auf das Touchscreendisplay das Gefühl einer mechanischen Taste zu vermitteln. Dies ist insbesondere dann ein Zugewinn an Komfort, wenn das Gerät über keinerlei mechanische Tastatur verfügt, sondern nur über einen Touchscreen, wie beispielsweise bei verschiedenen mobilen Telefonen oder Kleinstcomputern. In diesen Anwendungen steht der Komfort der Bedienung im Vordergrund, sicherheitstechnische Vorteile werden durch den Touchscreen mit taktilem Feedback bei den bekannten Geräten nicht erreicht.

Bei medizintechnischen Geräten steht jedoch der Sicherheitsaspekt im Vordergrund.

Der Erfindung liegt deshalb die Aufgabe zugrunde, ein medizintechnisches Gerät mit Touchscreen derart vorteilhaft weiterzubilden, dass die Sicherheit des medizintechnischen Geräts und der Bedienkomfort erhöht werden.

Die Lösung dieser Aufgaben erfolgt erfindungsgemäß durch ein medizintechnisches Gerät mit den Merkmalen des Anspruchs 1 und durch ein Verfahren mit den Merkmalen des Anspruchs 8. Vorteilhafte Ausführungsformen sind Gegenstand der Unteransprüche.

Unter taktilen Signalen werden Verformungen, also dauerhafte oder alternierende Änderungen der Ausdehnung einer Sache verstanden.

Die Erfindung beruht auf einem medizintechnischen Gerät, insbesondere eine Dialysemaschine, mit mindestens einem Touchscreen, wobei der Touchscreen über zwei redundante Sensoren zur Detektion der Position eines Fingerdrucks auf dem Touchscreen verfügt, und wobei zumindest einer der Sensoren eine Vielzahl von Piezoelementen umfasst und das medizinische Gerät dazu eingerichtet ist, zu erkennen, ob die optische Anzeige des Touchscreens funktioniert und im Falle, dass erkannt wird, dass die optische Anzeige des Touchscreens nicht funktioniert, die Piezoelemente derart ansteuert , dass Teilflächen des Touchscreens unterschiedliche taktile Eigenschaften haben.

Weiterhin beruht die Erfindung auf einem Verfahren zur Anzeige und Eingabe von Informationen an einem medizintechnischen Gerät mit einem Touchscreen mit zumindest zwei redundanten Sensoren, wobei die Position eines Fingerdrucks auf den Bildschirm mit beiden Sensoren bestimmt wird und erkannt werden kann, ob die optische Anzeige des Touchscreens funktioniert und im Falle, dass erkannt wird, dass die optische Anzeige des Touchscreens nicht funktioniert, die Piezoelemente derart angesteuert werden können, dass Teilflächen des Touchscreens unterschiedliche taktile Eigenschaften haben.

Piezoelemente nutzen den piezoelektrischen Effekt, um entweder durch Anlegen einer elektrischen Spannung eine Bewegung auszuführen, oder bei Einwirkung einer Kraft eine elektrische Spannung zu erzeugen. Piezoelement sind also gleichermassen als Kraftsensoren, sowie als Aktoren einrichtbar. Piezoelemente können bestimmte Kristalle (Piezokristalle) oder piezoelektrische Keramiken, also polykristalline Materialien sein.

Die Piezoelemente können matrixartig über die Touchscreenoberfläche verteilt sein und über entsprechende Ansteuerungen ortsauflösend individuell ansteuerbar sein.

Die Piezoelemente können auch an den Ecken oder den Seiten unterhalb des Displays kraftschlüssig mit dem Display verbunden sein.

In einer alternativen Ausführungsform können die Piezoelemente auch an bestimmten Positionen des Displays vermehrt angeordnet sein und an bestimmten anderen Positionen vermindert oder gar nicht vorhanden sein. So kann an bestimmten Positionen des Touchscreens eine verbesserte Auflösung des taktilen Signals erreicht werden, ohne die Gesamtzahl der Piezoelemente zu erhöhen.

In einer Ausführungsform werden die Piezoelemente dadurch gebildet, in dem eine im Wesentlichen lichtdurchlässige piezoelektrische Schicht an jeweils gegenüberliegenden Seiten von im Wesentlichen lichtdurchlässigen Elektroden kontaktiert wird. Hierbei verlaufen eine Vielzahl von Elektroden an der Oberseite und der Unterseite der piezoelektrischen Schicht vorzugsweise in regelmäßigen Abständen zueinander orthogonal und vorzugsweise über das gesamte Display.

Ein Piezoelement wird in der Aufsicht als Überschneidung von einer auf der Oberseite der piezoelektrischen Schicht verlaufenden Elektrode mit einer Elektrode, die an deren Unterseite verläuft, und dem dazwischen liegenden piezoelektrischen Material gebildet.

Die relativ zur piezoelektrischen Schicht oben liegenden Elektroden bilden dabei ein Muster aus vorzugsweise gleichbreiten Linien, die zueinander parallel und vorzugsweise äquidistant sind, mit einem Abstand, der vorzugsweise der Linienbreite entspricht. Gleichermaßen sind die an der relativ zur piezoelektrischen Schicht unten liegenden Elektroden angeordnet. In der Aufsicht bilden beide Elektrodenebenen ein gleichmäßiges Gitter, wobei jede Überschneidung von über und unter der piezoelektrischen Schicht verlaufenden Elektroden ein individuell anzusteuerndes bzw. auszulesendes Piezoelement bildet.

Die Multiplikation der Anzahl der Elektroden an der Oberseite der piezoelektrischen Schicht mit der Anzahl der Elektroden an der Unterseite der piezoelektrischen Schicht ergibt die Anzahl der piezoelektrischen Elemente.

Ein bestimmtes Piezoelement ist durch die Auswahl jeweils einer Elektrode an der Oberseite und einer Elektrode an der Unterseite der piezoelektrischen Schicht ansteuerbar bzw. auslesbar. Durch das Anlegen einer Spannungsdifferenz an jeweils einer Elektrode an der Oberseite und einer Elektrode an der Unterseite der piezoelektrischen Schicht wird das piezoelektrische Material zwischen diesen beiden Elektroden zu einer Deformation angeregt.

Auf die gleiche Art kann die Spannung, die über einem piezoelektrischen Element abfällt, an den dieses piezoelektrische Element kontaktierenden Elektroden abgegriffen werden. Nachteilig erweist sich bei dieser Ausführung, dass die einzelnen Piezoelemente nicht gleichzeitig unabhängig voneinander ansteuer- bzw. auslesbar sind. Eine einzige Elektrode bestimmt das einseitige Potential einer Vielzahl von Piezoelementen.

In einer besonders bevorzugten Ausführungsform ist daher eine Vielzahl von lichtdurchlässigen Elektroden, die bevorzugt quadratförmig ausgeführt sind, an der Oberseite der piezoelektrischen Schicht angeordnet. Jede dieser Elektroden ist durch eine eigene Steuerleitung ansteuer- und auslesbar. Die Unterseite der piezoelektrischen Schicht trägt eine einzige lichtdurchlässig Elektrode, die über die gesamte Fläche der piezoelektrischen, lichtdurchlässigen Schicht verläuft, und deren Potential durch einen einzige Steuerleitung ansteuer- und auslesbar ist. Ein Piezoelement entsteht bei dieser Ausführungsform zwischen einer Elektrode auf der Oberseite der piezoelektrischen Schicht und der gemeinsamen Elektrode auf der Unterseite der piezoelektrischen Schicht. Diese Ausführungsform ermöglicht die individuelle und gleichzeitige Ansteuerbarkeit und Auslesbarkeit jedes einzelnen Piezoelements.

Die Fläche der Elektroden an der Oberseite der piezoelektrischen Schicht kann sich an der Berührfläche eines menschlichen Fingers orientieren (ca. 1cm²). Es ist auch möglich, die Elektroden an der Oberseite der piezoelektrischen Schicht in ihrer Form und Größe individuell zu gestalten, um beispielsweise einer bevorzugten optischen Anzeige angepasst zu werden. Beispielsweise kann eine an einer bestimmten Stelle des Touchscreens immer vorkommende große Bedienfläche (z.B. "enter") mit einem einzigen entsprechend größeren Piezoelement überbaut sein, während an anderer Stelle kleinere Elektroden vorhanden sind.

Als Material für die Piezoelemente kommen natürliche Kristalle wie Berlinit, Rohrzucker, Quarz, Rochellesalz, Topaz oder Turmaline, künstliche Kristalle wie Galliumorthophosphat oder Langasit, Keramiken oder Polymere in Frage. Grundsätzlich ist jedes Material geeignet, das sich aufgrund einer angelegten Spannung verformt.

Die Elektroden können aus einem Metall oder einem Halbleiter bestehen, vorzugsweise sind sie als Indiumzinnoxid (ITO) Elektroden, die für sichtbares Licht weitgehend transparent sind, ausgeführt.

Im Folgenden werden die Ausführungsformen des Touchscreens mit taktilem Feedback am Beispiel einer Dialysemaschine beschrieben. Dem Fachmann ist klar, dass die beschriebenen Ausführungsformen ohne Einschränkung auf jedes medizintechnische Gerät übertragbar sind.

In einer Ausführungsform ist die Vorrichtung dazu ausgestaltet, die durch Druckeinwirkung erzeugte Spannung jedes Piezoelements auszulesen. Die Piezoelemente arbeiten in dieser Ausführungsform als Kraftsensoren. Dadurch wird ein Fingerdruck auf eine entsprechende Stelle des Touchscreens detektiert. Es kann vorkommen, dass die Eigenschaft des Touchscreens, Berührungen positionsaufgelöst zu detektieren, eingeschränkt oder nicht mehr vorhanden ist, z.B. durch einen technischen Defekt. In diesem Fall kann durch Auslesen der durch einen Fingerdruck erzeugten Spannungen der Piezoelemente eine alternative, zu der ohnehin vorhandenen Vorrichtung des Touchscreens redundante, Möglichkeit geschaffen werden, die Bedienereingabe positionsaufgelöst zu detektieren.

Vorstellbar ist, dass bei jedem Fingerdruck auf den Touchscreen die Signale der Vorrichtung des Touchscreens, die die Position des Fingers auf dem Touchscreen ermittelt (im Folgenden Touchscreen-Fingersensor genannt), mit den entsprechenden Spannungen der Piezoelemente verglichen werden, um so die Funktionalität beider Sensorvorrichtungen zu überprüfen.

Beispielsweise kann es vorkommen, dass die Funktionalität des Touchscreens, Berührungen positionsaufgelöst zu detektieren, defekt oder eingeschränkt ist, die Funktionalität der Piezoelemente aber gegeben ist. Bei einer Bedienereingabe durch einen Fingerdruck auf den erfindungsgemäßen Touchscreen erzeugen die Piezoelemente, auf die der Bediener durch einen Fingerdruck Kraft einwirken lässt, eine elektrische Spannung. Eine Auswerte- und Steuervorrichtung, die die Signale des Touchscreen-Fingersensors mit den Signalen der Piezoelemente vergleicht, erkennt in diesem Fall das Ausbleiben der Sensorsignale des Touchscreen-Fingersensors und schließt auf einen Defekt bzw. auf einen Fehlerfall. Analog kann ein Defekt der Funktionalität bzw. auf einen Fehlerfall der Piezoelemente erkannt werden, wenn die Auswerte- und Steuervorrichtung Signale des Touchscreen-Fingersensors detektiert, aber keine Signale der Piezoelementmatrix. In beiden Fällen kann die Auswerte- und Steuervorrichtung das Vorhandensein eines Defekts dem Bediener und/oder einer weiteren Steuervorrichtung der Dialysemaschine anzeigen. Diese Anzeige kann ein Steuersignal und/oder ein optisches, taktiles oder akustisches Signal sein. Die Steuerung der Dialysemaschine kann bei Auftreten eines Defekts Maßnahmen ergreifen, z.B. eine laufende Dialysebehandlung kontrolliert beenden, oder aber zu Ende führen und eine weitere Behandlung erst nach Beseitigung des Defekts erlauben.

Diese redundante Eingabemöglichkeit ermöglicht weitaus flexiblere Eingabemöglichkeiten als redundante mechanische Schalter, deren Anzahl und Position im Betrieb nicht veränderbar ist. Durch die Flexibilität des Touchscreens mit taktilem Feedback kann die Bedienbarkeit der Dialysemaschine im Fehlerfall uneingeschränkt aufrechterhalten werden. Das bedeutet für den Patienten eine wesentliche Verbesserung für den Fall, dass der Touchscreen-Fingersensor ausfällt, da die Bedienbarkeit der Dialysemaschine durch die redundante Eingabemöglichkeit uneingeschränkt weiterhin vorhanden sein kann. Eine Unterbrechung der aktuellen Dialysebehandlung ist somit nicht zwingend notwendig, was den Komfort und die Sicherheit erhöht.

In einer weiteren Ausführungsform kann die Piezoelementmatrix auch dazu genutzt werden, die Fingerdrücke mehrerer Finger gleichzeitig zu erkennen. Im Stand der Technik sind Touchscreens mit Multitouchfunktionalität bekannt, die auf einer speziellen kapazitiven Touchscreentechnologie aufbauen.

Bei konventionellen kapazitiven Touchscreendisplays bildet ein sich der Displayoberfläche nähernder Finger mit einer im Wesentlichen für sichtbares Licht durchsichtigen Beschichtung (z.B. aus ITO) auf dem Touchscreen eine Kapazität. Über der leitfähigen Beschichtung ist in der Regel eine kratzfeste Folie oder eine Glasabdeckung appliziert, um die Beschichtung vor Abnutzung zu bewahren und den Touchscreen robust auszugestalten. Diese Folie bzw. die Glasabdeckung wirken hierbei als Dielektrikum für den sich bildenden Plattenkondensator, dessen eine Platte die leitfähige Beschichtung darstellt und dessen andere Platte der sich nähernde bzw. aufliegende Finger bildet. Wesentlich für die Funktion kapazitiver Touchscreens ist daher, dass der berührende Gegenstand Ladung aufnehmen kann, was bei einem menschlichen Finger der Fall ist. Die Ecken der leitfähigen Beschichtung sind mit Wechselspannungsquellen verbunden. Bei Berührung des Touchscreen fließen Ströme von diesen Wechselspannungsquellen über die leitfähige Schicht und den Kondensator, der durch den Finger und die leitfähige Schicht gebildet wird. Hierbei bildet die Wegstrecke von der jeweiligen Wechselspannungsquelle zur Berührstelle des Fingers einen elektrischen Widerstand, dessen Größe von der spezifischen Leitfähigkeit der Beschichtung und der Länge der Wegstrecke abhängt. Es ergeben sich also zur Länge der Wegstrecke proportionale Stromhöhen, die von einer Auswerteschaltung erfasst werden und der aktuellen Fingerposition zugeordnet werden können.

Diese verbreitete und preiswerte Ausführungsart kapazitiver Touchscreens kann nicht die Positionen zweier berührender Finger bestimmen, weil die Stromsignale bei zwei Berührstellen nicht eindeutig den Positionen der Berührungen zugeordnet werden können. Es sind zwar aus dem Stand der Technik Touchscreens mit Multitouchfunktionalität bekannt, gängige Dialysemaschinen sind aber nicht mit Touchscreens mit Multitouchfunktionalität ausgerüstet.

Durch die zusätzliche Piezoelementschicht kann eine solche Funktionalität ebenfalls umgesetzt werden. Da jedes Piezoelement individuell abgefragt werden kann, werden auch gleichzeitige Fingerdrücke auf verschiedenen Piezoelementen detektiert. Dies kann z.B. dazu benutzt werden, um mit zwei Fingern ein Intervall von Werten z.B. auf einer Werteskala aufzuspannen, um z.B. bequem Unter- und Obergrenzen eines Parameters einzugeben. Ebenfalls vorstellbar ist, dass mit der Bewegung zweier Finger auf dem Touchscreen eine vergrößerte oder verkleinerte Darstellung der Touchscreenanzeige unterhalb der Fingerbewegung ausgelöst wird.

In einer weiteren Ausführungsform sind die Piezoelemente dazu ausgestaltet, durch Anlegen einer elektrischen Steuerspannung eine Bewegung, vorzugsweise senkrecht zur Oberfläche des Touchscreens, auszuführen. Die elektrische Steuerspannung kann über die Zeit veränderlich, oder für eine Zeitspanne gleich sein.

Erfindungsgemäß können hierdurch Teile des Touchscreens individuell in ihren taktilen Eigenschaften verändert werden. Die Veränderungen umfassen im Wesentlich Vertiefungen oder Erhöhungen von Teilflächen des Touchscreens gegenüber den Umgebungsflächen, Vibration von Teilflächen und Änderung der Rauhigkeit (glatt, rau) von Teilflächen des Touchscreens gegenüber Umgebungsflächen. Alle Änderungen von taktilen Eigenschaften, die Teilflächen des Touchscreens betreffen, können bei entsprechender Auslegung der erfindungsgemäßen Vorrichtung und der erfindungsgemäßen Verfahren auch auf die Gesamtfläche des Touchscreens anwendbar sein.

Es kann beispielsweise an ein oder mehrere Piezoelemente der Matrix eine elektrische Gleichspannung angelegt werden, worauf sich diese in einer Dimension verlängern oder verkürzen (in Abhängigkeit der Polung der anliegenden Gleichspannung) und zwar so lange, solange die Gleichspannung anliegt. Diese Verkürzung oder Verlängerung des einen oder der mehreren Piezoelemente wird von einem Bediener, der einen Finger an der über den betroffenen Piezoelementen befindlichen Stelle auflegt, als Vertiefung oder Erhöhung gefühlt.

Auf diese Weise kann eine Stelle auf dem Touchscreen geschaffen werden, wie beispielsweise ein Eingabefeld, das gegenüber der Umgebungsfläche als erhöhte Drückfläche erscheint. Als Eingabefeld ist eine Teilfläche des Touchscreen definiert, die bei Berührung durch einen Bediener zu einer eindeutigen Eingabe von Informationen führt.

Beispielsweise kann eine 10er Tastatur optisch dargestellt werden. Durch erhöhte Stellen über der jeweiligen angezeigten Ziffer mit ansonsten nicht erhöhten Trennlinien zwischen den Eingabefeldern kann somit der Eindruck einer mechanischen Taste geschaffen werden.

Zur Unterstützung des taktilen Effekts und zur Simulation mechanischer Taster oder Schalter kann es vorgesehen sein, dass die Auswerte- und Steuervorrichtung nach einer durch die Sensoren des Touchscreens festgestellten Berührung eines Fingers des Bedieners auf ein erhöhtes Eingabefeld, die Piezoelemente, die dieses Eingabefeld bilden, derart ansteuert, dass sie in der Folge eine Vertiefung oder eine ebene Fläche bilden. Hierzu wird die Ansteuerspannung der entsprechenden Piezoelemente nach der festgestellten Berührung umgepolt oder abgestellt. Diese Umpolung oder das Abstellen der Steuerspannung kann plötzlich erfolgen oder aber sich langsamer vollziehen.

Vorstellbar ist ebenfalls, dass die Spannung über den Piezoelementen auch im angesteuerten Zustand überwacht wird. Somit kann eine mechanische Krafteinwirkung durch einen Fingerdruck detektiert werden. Je stärker der Fingerdruck auf das Piezoelement ist, umso stärker ist die Modulation der Ansteuerspannung durch die von dem Piezoelement erzeugte Spannung. In der Praxis kann eine Veränderung des Gleichspannungsanteils der Steuerspannung durch eine Krafteinwirkung auf das Piezoelement beobachtet werden. Die Piezoelemente arbeiten in dieser Ausführungsform gleichzeitig als Kraftsensoren und als Aktoren.

Diese Information kann dazu benutzt werden, um nicht beabsichtigte Berührungen des Touchscreens von einer gewollten Bedienereingabe zu unterscheiden. Eine ungewollte Berührung des Touchscreens löst im Zweifelsfall ein Signal der Sensoren des Touchscreens aus, während die Spannungen über den betroffenen Piezoelementen des Bedienfelds nicht, oder nur wenig moduliert werden. Eine gewollte Berührung erfolgt mit einer bestimmten Krafteinwirkung durch den Finger des Bedieners, was sowohl ein Signal der Touchscreensensoren als auch eine bestimmte Modulation der Spannungen über den betroffenen Piezoelementen bewirkt, die größer als ein zuvor festgelegter Grenzwert ist. Bei Überschreiten des Grenzwertes wird demnach eine gewollte Eingabe detektiert, wonach die Bedienfelder in schon beschriebener Weise von einem erhöhten in einen vertieften Zustand wechseln können. Somit wird sichergestellt, dass zur Bedienereingabe ein bestimmter Kraftaufwand notwendig ist. Darüber hinaus wird damit auch das Verhalten mechanischer Schalter oder Taster realistischer nachgebildet.

Die Erfindung sieht vor, dass bei Ausfall der optischen Anzeige des Touchscreens eine Bedienbarkeit über die Piezoelemente sichergestellt wird. Wird beispielsweise festgestellt, dass die optische Anzeige des Touchscreens defekt ist, beispielsweise durch eine Bedienereingabe über eine entsprechende alternative Eingabemöglichkeit, z.B. eine "Display defekt" Taste, oder über die Abfrage von geeigneten Sensoren, z.B. von lichtempfindlichen Bauteilen (Fotodioden, Fototransistoren, etc.), die die Hintergrundbeleuchtung des Displays überwachen und im Falle eines Ausfalls der Hintergrundbeleuchtung dem Steuergerät eine entsprechende Meldung übergeben, können die Piezoelemente dermaßen angesteuert werden, dass sie gleichzeitig als Eingabe- und Ausgabevorrichtung zu verwenden sind.

In diesem Fall wird die Displayoberfläche in mehrere Teilflächen mit unterscheidbaren taktilen Eigenschaften unterteilt, so dass sie von einem Bediener fühlbar unterscheidbar sind. In einer Ausführungsform wird jeder Teilfläche eine bestimmte, dem Bediener bekannte und/oder durch ein geeignetes Mittel dokumentierte Aktion zugeordnet. Zum Beispiel kann ein Dokument vorhanden sein, das zeigt, welche Teilflächen des Displays mit welcher taktilen Eigenschaft verbunden sind, und welche Aktion ausgeführt wird, wenn diese Teilfläche durch Finger- oder Handdruck des Bedieners ausgewählt wird. Es kann beispielsweise dokumentiert sein, dass die Touchscreenoberfläche in vier Teile unterteilt ist, deren Lage durch entsprechende taktile Eigenschaften und/oder auch Positionen auf dem Touchscreen charakterisiert ist. So kann z.B. eine Teilfläche mit der taktilen Eigenschaft "erhöht" und "rechts unten" charakterisiert sein und dieser Teilfläche die Aktion "Dialysebehandlung beenden" zugewiesen sein. Eine andere Teilfläche kann analog mit der taktilen Eigenschaft "vibrierend" und "links oben" charakterisiert sein und mit der Aktion "Dialysebehandlung fortsetzen" verbunden sein. Im Falle eines Ausfalls der optischen Anzeige des Touchscreens kann somit eine zumindest eingeschränkte Bedienbarkeit der Dialysemaschine sichergestellt sein, um z.B. eine Dialysebehandlung gefahrlos zu beenden oder im Ermessen der Bedienperson (Arzt) gefahrlos fortzusetzen.

Die erfindungsgemäße Vorrichtung und die erfindungsgemäßen Verfahren bieten also eine zusätzliche Sicherheit bei Dialysemaschinen, in dem sie bei Ausfall der optischen Anzeige des Touchscreens eine zumindest reduzierte Bedienbarkeit der Dialysemaschine sicherstellen.

In einer weiteren Ausführungsform der Erfindung kann es vorgesehen sein, dass nur Bedienflächen, die zur Benutzereingabe vorbestimmt sind, erhöht sind. Beispielsweise kann bei der Eingabe von Zahlenwerten, die sich nur in einem bestimmten Intervall bewegen dürfen, die Eingabe derart gestaltet werden, dass in der Reihenfolge der Eingabe der Ziffern jeweils nur erlaubte Bedienfelder, nämlich jene, die plausiblen bzw. erlaubten Ziffern zugeordnet sind, erhöht sind. Ist eine Eingabe zum Beispiel nur innerhalb des Intervalls der Zahlen von einschließlich 0 bis einschließlich 50 erlaubt, so sind bei der Eingabe der ersten Ziffer die Bedienfelder mit den Ziffern 0 bis 5 und bei der Eingabe der zweiter Ziffer die Bedienfelder mit den Ziffern 0 bis 9 bzw. nur die Ziffer 0 (wenn die erste Ziffer 5 lautete) erhöht. Unterstützt werden kann diese taktile Kennzeichnung erlaubter Bedienfelder durch entsprechende optische Darstellung und akustische Signale. So können erlaubte Bedienfelder in einer anderen Farbe oder Intensität als nicht erlaubte Bedienfelder dargestellt werden und bei Fehleingabe ein akustisches Signal ausgegeben werden.

Es kann vorgesehen sein, dass die Bedienfelder, die nicht zur Eingabe vorgesehen sind, durch die Piezoelemente vertieft werden.

Es kann stattdessen vorgesehen sein, dass die Bedienfelder, die zur Eingabe vorgesehen sind, durch die Piezoelemente vertieft werden und die Bedienfelder, die nicht zur Eingabe vorgesehen sind, erhöht werden.

Es kann ebenfalls vorgesehen sein, dass bei einem Fingerdruck auf nicht zur Eingabe vorgesehene Bedienfelder diese Bedienfelder vibrieren, um den Bediener auf die Fehleingabe taktil aufmerksam zu machen.

Diese Vibration wird durch die Ansteuerung der betroffenen Piezoelemente mit Wechselspannung erzeugt, wodurch die Piezoelemente mit der Frequenz der Ansteuerspannung vibrieren. Die Vibrationsfrequenz liegt vorteilhaft in Bereich von unter 100Hz und über 30Hz, um vom Bediener als Vibration erkannt zu werden. Eine Vibration mit dieser Frequenz und mit der durch die Piezoelemente eingeschränkten möglichen Amplitude und der in der Praxis sehr kleinen Fläche führt nicht zu einer hörbaren Schallabstrahlung und wird demnach nur gefühlt.

In einer weiteren Ausführungsform können die Piezoelemente derart angesteuert werden, dass sie mit einer hohen Frequenz vibrieren. Eine Vibration mit hoher Frequenz, vorzugsweise im Bereich über 20kHz, wird vom Bediener als raue Oberfläche gefühlt. Vorteilhaft bei einer Vibrationsfrequenz über 20kHz ist, dass die mit der Vibration verbundene Schallabstrahlung für den Menschen unhörbar ist. Auf diese Weise können sich Bedienflächen des Touchscreens von anderen Flächen oder Bedienflächen des Touchscreens fühlbar unterscheiden.

Eine weitere Ausführungsform betrifft die Verwendung von mindestens drei, bevorzugt aber vier Aktoren, bevorzugt Piezoelementen, die sich an den Rändern, oder den Ecken unterhalb des Touchscreens befinden und diesen dort formschlüssig lagern. Derart ausgestaltet kann das gesamte Display durch die Aktoren bewegt werden. Ebenso kann ein Fingerdruck auf das Display durch Abfrage der Piezoelementspannung oder alternativer Drucksensoren detektiert werden. Eine Krafteinwirkung eines Fingerdrucks auf das Display verteilt sich positionsabhängig auf die an den Rändern oder Ecken liegenden Kraftsensoren. Durch einen Vergleich der Sensorsignale können die Position und die Stärke eines Fingerdrucks bestimmt werden.

Ohne Einschränkung können alle zuvor beschriebenen Ansteuerungen und Auslesevorgänge der Piezoelemente auch miteinander kombiniert werden. So ist beispielsweise eine erhöhte, vibrierende Fläche dadurch realisierbar, dass die entsprechenden Piezoelemente mit einer Wechselspannung mit einem Gleichanteil angesteuert werden. Ebenso ist eine vertiefte, vibrierende Fläche durch eine Ansteuerung der entsprechenden Piezoelemente mit einer Wechselspannung mit einem Gleichanteil, der nun gegenteiliges Vorzeichen hat, möglich.

Im Folgenden wird die vorliegende Erfindung unter Bezugnahme auf die beigefügten Figuren anhand von Ausführungsbeispielen beschrieben. In der Zeichnung bezeichnen identische Bezugszeichen identische oder funktionsgleiche Elemente.

Es gilt:
Figur 1 zeigt eine Ausführungsform eines erfindungsgemäßen medizintechnischen Geräts, das als Dialysemaschine ausgeführt ist
Figur 2a zeigt einen Aufbau eines Touchscreens mit Piezoelementen und symbolisierter Ansteuerung als erste Ausführungsform eines Touchscreens eines erfindungsgemäßen medizintechnischen Geräts, wie z.B. nach Fig. 1
Figur 2b zeigt einen alternativen Aufbau eines Touchscreens mit einer Vielzahl von Piezoelementen und symbolisierter Ansteuerung als zweite Ausführungsform eines Touchscreens eines erfindungsgemäßen medizintechnischen Geräts, wie z.B. nach Fig. 1
Figur 2c zeigt einen weiteren alternativen Aufbau eines Touchscreens mit aktorischer Lagerung als dritte Ausführungsform eines Touchscreens eines erfindungsgemäßen medizintechnischen Geräts, wie z.B. nach Fig. 1
Figur 3 zeigt ein einzelnes Piezoelement mit Elektroden eines Touchscreens nach Fig. 2a
Figur 4 zeigt ein Beispiel für eine Displayanzeige eines Touchscreen, wie z.B. nach Figur 2a, an einem erfindungsgemäßen medizintechnischen Gerät mit taktiler Zusatzinformation

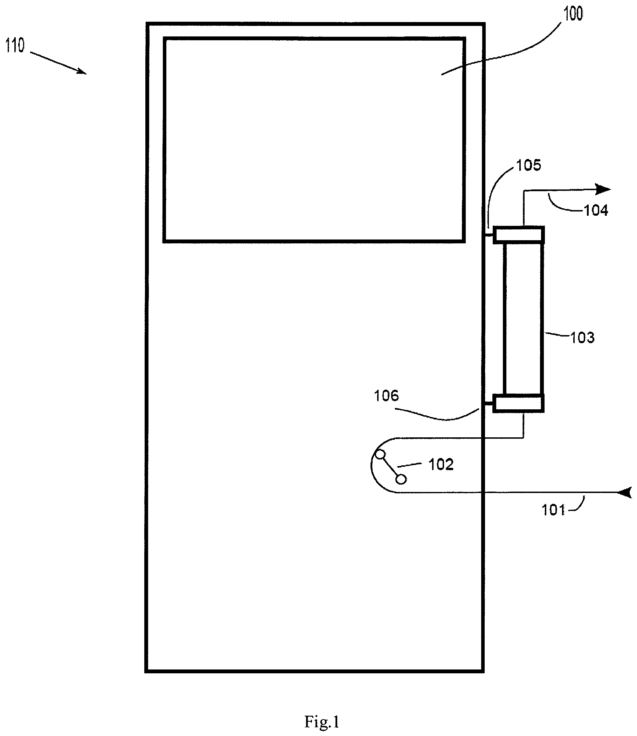

In Figur 1 ist eine Ausführungsform eines erfindungsgemäßen medizintechnischen Gerätes 110 als Dialysemaschine mit einem Touchscreendisplay mit taktilem Feedback 100 ausgeführt schematisch dargestellt. Die Dialysemaschine 110 zeigt andeutungsweise Teile eines extrakorporalen Blutkreislaufs mit einer arteriellen Blutleitung 101, die Blut eines Patienten (nicht dargestellt) ableitet. Die Blutpumpe 102 fördert das Blut durch einen Dialysefilter 103, der mit einer semipermeablen Membran ausgestattet ist, die den extrakorporalen Blutkreislauf von einem Dialysatkreislauf semipermeabel trennt. Über die venöse Leitung 104 wird das behandelte Blut dem Patienten zurückgegeben. Über die Dialysatleitungen 105 und 106 wird Dialysat durch den Dialysefilter 103 gepumpt, wo es über die semipermeable Membran des Dialysefilters 103 zu einem diffusiven Stoffaustausch mit dem Blut des Patienten kommt. Wird zusätzlich ein Druckgradient von der Blutseite des Dialysefilters zur Dialysatseite des Patienten aufgebaut, wird Plasmawasser aus dem Blut in das Dialysat abgepresst. Das Blut des Patienten kann so entwässert werden. Das Dialysat wird in der Hämodialysemaschine 110 hergestellt und nach Gebrauch verworfen.

Die Dialysemaschine in Figur 1 ist als Hämodialysemaschine ausgeführt. Ohne Einschränkung sind die erfindungsgemäße Vorrichtung und das erfindungsgemäße Verfahren bei allen medizintechnischen Geräten anwendbar, bei denen Informationen und Bedienereingaben auf einem Touchscreendisplay angezeigt bzw. entgegengenommen werden. Dies umfasst ausdrücklich und nicht abschließend auch Blutbehandlungsvorrichtungen wie zum Beispiel Geräte zur automatischen Peritonealdialyse, ebenso wie Vorrichtungen zur Hämofiltration, Hämodiafiltration, Plasmapherese oder ähnliche Verfahren.

Im Rahmen einer Dialysebehandlung sind in der Regel umfangreiche Bedieneingaben in eine Dialysemaschine vorzunehmen, bzw. Informationen, beispielsweise das gewählte Dialyseverfahren oder Informationen den Patient oder den Behandlungsverlauf betreffend, anzuzeigen. Ein wesentliches Sicherheitsmerkmal einer Dialysebehandlung ist, dass die Bedieneingaben zu jeder Zeit sicher und eindeutig funktionieren. Fehlbedienungen oder der Ausfall der Bedienmöglichkeit können ernste Folgen für die Sicherheit und die Gesundheit des Patienten haben, wenn sie unbemerkt bleiben.

Durch die erfindungsgemäße Verwendung eines Touchscreens mit taktilem Feedback in Dialysemaschinen wird die Bedieneingabe in der beschriebenen Weise wesentlich sicherer und komfortabler.

Bislang sind die Möglichkeiten, bei Ausfall des Fingerdrucksensors des Touchscreens Bedieneingaben zu tätigen, auf mechanische Schalter oder Bedienmöglichkeiten, die sich an der Maschine, aber nicht auf dem Touchscreen befinden, beschränkt. Hierdurch kann es zu Beeinträchtigungen der Behandlung kommen, da nicht die volle Funktionalität des Touchscreens redundant durch mechanische Schalter nachgebildet werden kann, sondern allenfalls eine sehr eingeschränkte Bedienbarkeit ermöglicht wird.

Durch die Vorrichtung und das Verfahren wird eine alternative, flexible und eine zu dem Fingerdrucksensor des Touchscreens redundante Möglichkeit geschaffen, Bedieneingaben vorzunehmen. Bei entsprechender Auslegung der Vorrichtung ist die Bedienbarkeit im Fehlerfall des Fingerdrucksensors des Touchscreens nicht beeinträchtigt, und eine Behandlung kann planmäßig fortgesetzt werden.

Während die Behandlung bei einem medizintechnischen Gerät ohne redundante Sensoren zur Detektion der Position eines Fingerdrucks auf dem Touchscreen im Fehlerfall des Fingerdrucksensors des Touchscreens aus Sicherheitsgründen ab- bzw. unterbrochen werden sollte, kann bei einem medizintechnischen Gerät mit redundanten Sensoren zur Detektion der Position eines Fingerdrucks auf dem Touchscreen die Behandlung planmäßig weitergeführt und beendet werden, was eine wesentliche Verbesserung des Behandlungskomforts, der Sicherheit und des Behandlungseffekts bedeutet.

Darüber hinaus ergibt sich die Möglichkeit, die Bedieneingabe daraufhin zu überprüfen, ob sie beabsichtigt oder unbeabsichtigt sind, z.B. durch unbemerkte Berührung, in dem die Kraft ausgewertet wird, mit der auf eine Bedienfläche gedrückt wird. Auf diese Weise wird die Bediensicherheit erhöht und fehlerhafte Bedieneingaben verhindert.

Der Eingabekomfort kann wesentlich erhöht werden, in dem es möglich ist, Teile oder den gesamten Touchscreen mit taktilen Eigenschaften zu belegen. So kann dem Bediener durch ein taktiles Feedback signalisiert werden, ob die Bedienfläche, die er gerade berührt, zur Eingabe geeignet ist, z.B. dadurch, in dem diese Bedienfläche gegenüber der Umgebungsfläche erhöht ist. Fehlbedienungen oder Eingaben, die aus Sicherheitsgründen nicht zu einer Änderung der Behandlung führen, werden auf diese Weise auch taktil signalisiert, was der Bediener leichter bemerken kann, als nur bei einer optischen und/oder akustischen Warnung. Somit wird die Bediensicherheit eines medizintechnischen Gerätes durch die Erfindung weiter erhöht.

Weiterhin kann in schon beschriebener Weise die Möglichkeit der Multitouchfunktionalität, also dem Erkennen von Mehrfachberührungen zur gleichen Zeit an unterschiedlichen Positionen auf dem Touchscreen. Hierdurch können Komfortmerkmale, wie z.B. das Vergrößern von Darstellungen auf dem Touchscreen oder die Eingabe von Werteintervallen auf einer Werteskala komfortabel durch entsprechende Fingerbewegungen realisiert werden. Hierdurch erhöhen sich der Eingabekomfort und die Darstellungsqualität wesentlich.

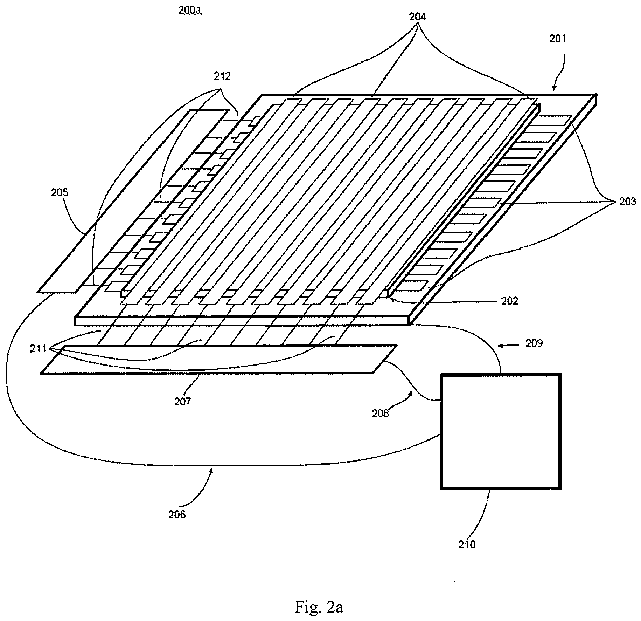

In Figur 2a ist eine Ausführungsform eines Touchscreens 200a mit taktilem Feedback für ein erfindungsgemäßes medizintechnisches Gerät dargestellt. Ein konventionelles Touchscreendisplay 201, wie aus dem Stand der Technik bekannt, ist vorzugsweise in kapazitiver Touchscreentechnologie ausgeführt.

Über dem Display 201 ist eine Anordnung aus den hier horizontal verlaufenden Elektroden 203, einer piezoelektrischen Schicht 202 und den vertikal verlaufenden Elektroden 204 angeordnet.

Horizontale und vertikale Elektroden können individuell und unabhängig voneinander über die beiden Steuervorrichtungen 205 und 207 mit einer Spannung versorgt und/oder die Spannung der Elektroden ausgelesen werden. Hierzu sind die Elektroden 204 über die leitenden Verbindungen 211 mit der ersten Steuervorrichtung 207 und die Elektroden 203 über die leitenden Verbindungen 212 mit der zweiten Steuervorrichtung 205 verbunden.

Aus Übersichtlichkeitsgründen stellt Figur 2a nur wenige Elektroden dar. In der Praxis kann die Elektrodenbreite sehr viel kleiner, beispielsweise 0.1mm oder weniger sein. Somit erhöht sich die Anzahl der Elektroden und damit die Auflösung der piezoelektrischen Schicht 202.

Über eine dritte Steuervorrichtung 210 werden sowohl der Touchscreen als auch die horizontalen und vertikalen Elektroden angesteuert und/oder ausgelesen. Hierzu ist die dritte Steuervorrichtung 210 über die Steuer- und Ausleseleitungen 206, 208 und 209 mit dem Touchscreen 201 und den beiden Steuervorrichtungen 205 und 207 verbunden. Die Steuer- und Ausleseleitungen 206, 208 und 209 sind in der Regel als Daten-Bus ausgeführt und bestehen aus mehreren signalführenden Leitungen, können aber auch Einzelleitungen sein.

Ein Piezoelement entsteht durch eine Überkreuzung einer vertikalen Elektrode 204 mit einer horizontalen Elektrode 203 mit dem zwischen den Kreuzungsflächen liegenden Teil der piezoelektrischen Schicht 202.

Soll dieses Piezoelement angesteuert werden, müssen die entsprechenden Elektroden mit jeweils einer Spannung beaufschlagt werden. Die Spannungsdifferenz der Einzelspannungen der Elektroden ist die Ansteuerspannung für dieses Piezoelement. Je nach Polung dieser Spannungsdifferenz verkürzt oder verlängert sich das angesteuerte Piezoelement in Richtung der beiden Elektroden.

Die Piezoelemente sind in der in Figur 2a dargestellten Ausführungsform nicht gleichzeitig individuell ansteuerbar oder auslesbar. Dennoch kann eine Reihe von benachbarten Piezoelementen gleichermaßen angesteuert werden, um z.B. ein erhöhtes Bedienfeld zu generieren.

Werden die Piezoelemente als Kraftsensoren benutzt, so ist es vorteilhaft, wenn einzelne Reihen von Piezoelementen konsekutiv in schneller Reihenfolge abgefragt werden. So kann für eine Zeitspanne t die Spannung zwischen einer der Elektroden 203 und allen Elektroden 204 von den Steuervorrichtungen 205, 207 und 210 bestimmt werden, um anschließend in gleicher Art die Spannung zwischen einer benachbarten Elektrode 203 und allen Elektroden 204 zu bestimmen. Auf diese Weise werden in der weiteren Folge konsekutiv die Spannungen zwischen allen Elektroden 203 und allen Elektroden 204 bestimmt. Dieser Vorgang wird ständig wiederholt und ermöglicht bei hinreichend kleiner Zeitspanne t eine quasi kontinuierliche Überwachung aller Piezoelemente auf Krafteinwirkung durch einen Fingerdruck. Die Zeitspanne t ist dann hinreichend klein, wenn die Abfrage sämtlicher Piezoelemente schneller geschieht, als mögliche Bewegungen eines Bedienerfingers.

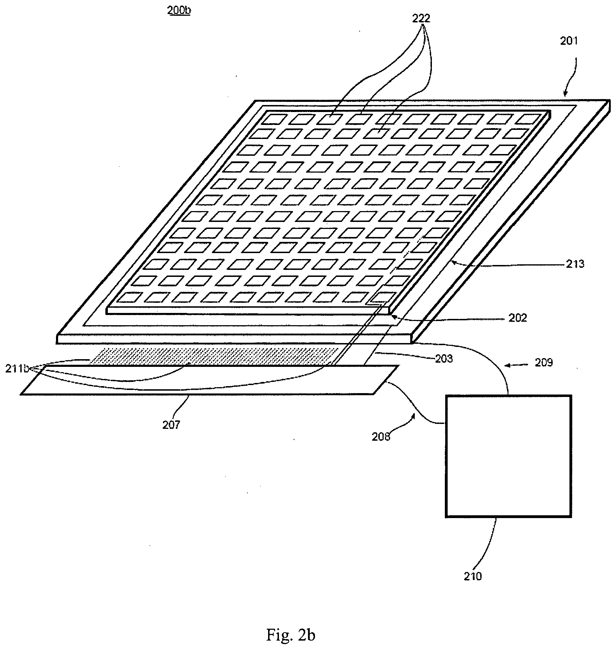

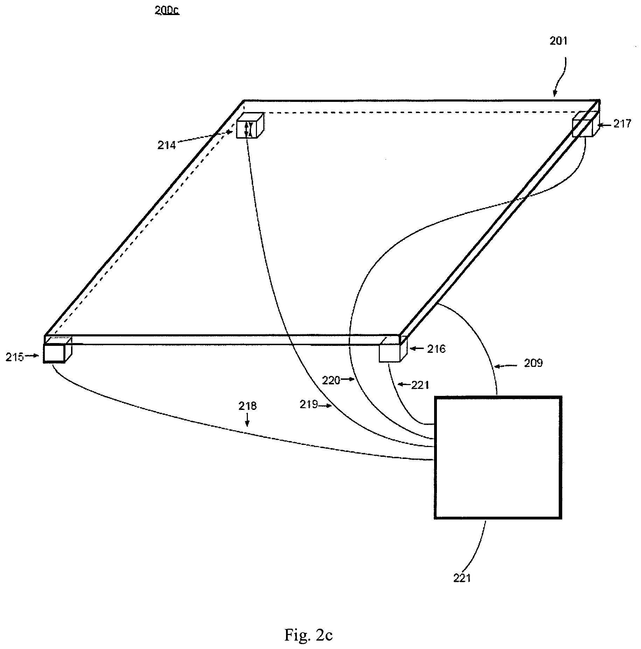

In den Figuren nicht dargestellt ist eine elektrisch isolierende Folie oberhalb der Vorrichtung 100a, 100b (Figur 2b) und 100c (Figur 2c). Diese Folie ist vorzugsweise dünn und elastisch. Neben der Eigenschaft, den Touchscreen mit taktilem Feedback elektrisch nach oben zu isolieren, bietet eine solche Folie auch eine hygienisch glatte Oberfläche.

Die Figur 2b zeigt eine alternative Ausführungsform eines Touchscreen 200b mit taktilem Feedback für ein erfindungsgemäßes medizintechnisches Gerät. Ein konventionelles Touchscreendisplay 201, wie aus dem Stand der Technik bekannt, ist vorzugsweise in kapazitiver Touchscreentechnologie ausgeführt.

Über dem Touchscreendisplay 201 ist eine lichtdurchlässige flächige Elektrode 213 angeordnet. Diese bildet eine gemeinsame Elektrode für alle Piezoelemente, die durch die piezoelektrische Schicht 202 und der Vielzahl von hier quadratischen Elektroden 222 auf der Oberseite der piezoelektrischen Schicht gebildet werden. Jede der Elektroden an der Oberseite ist durch eine individuelle Leitung 211b mit der Steuervorrichtung 207 elektrisch verbunden (ausgeführt sind der Übersichtlichkeit halber nur die Verbindungen zu zwei Piezoelementen, die restlichen Verbindungen sind durch unterbrochene Linien angedeutet), ebenso die gemeinsame Elektrode 213 durch die Steuerleitung 203.

Die Steuer- und Ausleseleitungen 208 und 209 sind in der Regel als Daten-Bus ausgeführt und bestehen aus mehreren signalführenden Leitungen, können aber auch Einzelleitungen sein.

Die Steuervorrichtung 210 empfängt Signale der ersten Steuervorrichtung 207 und sendet Signale an die Steuervorrichtung 207 über die Steuer- und Ausleseleitung 208. Über die Steuer- und Ausleseleitungen 209 ist der Touchscreen 201 mit der Steuervorrichtung 210 verbunden.

Bei der in der Figur 2b dargestellten Ausführungsform kann vorteilhaft jedes Piezoelement individuell und gleichzeitig mit allen anderen Piezoelementen angesteuert und abgefragt werden.

Ein weitere Ausführungsform für ein Touchscreen 100c mit taktilem Feedback zeigt die Figur 2c.

201 kennzeichnet hierbei ein konventionelles Touchscreendisplay, wie aus dem Stand der Technik bekannt, vorzugsweise in kapazitiver Touchscreentechnologie ausgeführt

Das Touchscreendisplay 201 ist in dieser Ausführungsform an den Ecken mit vier Piezoelementen 214, 215, 216 und 217 kraftschlüssig verbunden. Die Piezoelemente arbeiten hierbei als Aktoren und Kraftsensoren. Die Piezoelemente werden über die Signalleitungen 218, 219, 220 und 221 über die Steuervorrichtung 221 angesteuert und abgefragt. Durch die Ansteuerung der Piezoelemente kann sich das gesamte Display in Richtung der im Piezoelement 214 gezeichneten Pfeile bewegen.

In der in der Figur 2c gezeigten Ausführungsart können keine Teilflächen des Touchscreens 201 mit taktilen Eigenschaften belegt werden, sondern nur die gesamte Fläche des Touchscreens. Dennoch ist es möglich, einem Benutzer ein ortsaufgelöstes taktiles Feedback zu geben.

Hierzu wird, wenn ein Fingerdruck von dem Fingerdrucksensor des Touchscreens und der Steuervorrichtung 221 detektiert wird, unmittelbar entschieden, ob an der Stelle, wo der Finger den Touchscreen berührt, z.B. eine Erhebung (oder eine Vertiefung bzw. Vibration) gefühlt werden soll. Wenn das der Fall ist (der Bediener drückt z.B. auf eine dargestellte Bedienfläche), werden die Piezoelemente 214 bis 217 derart angesteuert, dass sich das gesamte Display erhebt. Wandert der Bedienfinger nun zu einer Stelle, der eine Vertiefung, eine Vibration oder eine Stelle innerhalb der normalen Benutzerebene zugeordnet ist, werden die Piezoelemente 214 bis 217 derart angesteuert, dass sich das gesamte Display entsprechend bewegt. Da der Benutzer normalerweise nur einen Finger zur Eingabe von Informationen verwendet, entsteht somit der Eindruck eines taktilen Feedbacks in Form von positionsaufgelösten taktilen Eigenschaften des Touchscreens.

Diese Ausführungsform kann auch zur Detektion eines Fingerdrucks verwendet werden. Drückt ein Bediener eine Stelle des Displays, verteilt sich die Kraft umgekehrt proportional zur Entfernung zu den einzelnen Piezoelementen 214 bis 217 auf diese. Werden die durch die Krafteinwirkung ausgelösten elektrischen Spannungen der einzelnen Piezoelemente durch die Steuereinheit 221 gemessen und ausgewertet, kann somit die Position des Fingerdrucks bestimmt werden.

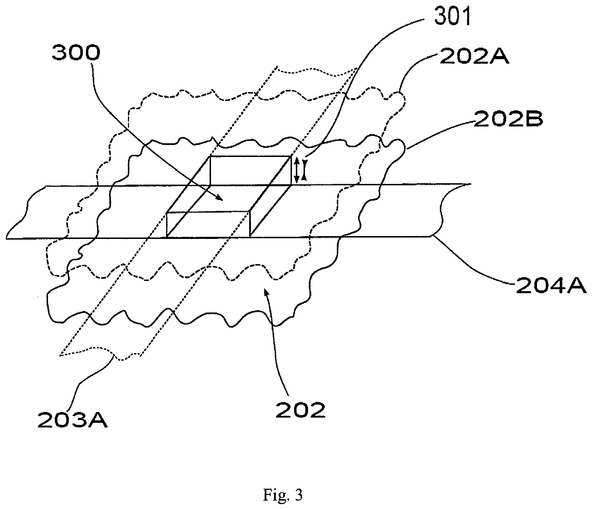

Die Figur 3 zeigt zur besseren Veranschaulichung ein einzelnes Piezoelement 300, das analog der Figur 2a durch die Überkreuzung der Elektrode 203A mit der Elektrode 204A und dem zwischen den Kreuzungsflächen liegenden Teil der piezoelektrischen Schicht 202 gebildet wird. Hierbei stellt die gestrichelt dargestellte Begrenzungsfläche 202A einen Ausschnitt der oberen Begrenzungsfläche der piezoelektrischen Schicht 202, die durchgezeichnete Begrenzungsfläche 202B einen Ausschnitt der unteren Begrenzungsfläche der piezoelektrischen Schicht 202 dar.

Die gestrichelt gekennzeichnete obere Elektrode 203A verläuft hierbei oberhalb von 202A und berührt diese Grenzfläche, so dass elektrischer Kontakt hergestellt ist. Analog verläuft die untere Elektrode 204A unterhalb von 202B und berührt diese Grenzfläche.

Über die beiden Elektroden 202A und 203A kann sowohl eine Differenzspannung an das Piezoelement 300 angelegt werden, als auch eine Spannung, die über dem Piezoelement 300 abfällt, ausgelesen werden, beispielsweise bei durch äußeren Druck hervorgerufene Verformung des Piezoelements 300.

Eine Verformung des Piezoelements 300 erfolgt auch durch eine über die beiden Elektroden 202A und 203A angelegte Differenzspannung in Richtung der Elektroden 202A und 203A, wie durch die beiden Pfeile 201 in Figur 3 gezeigt.

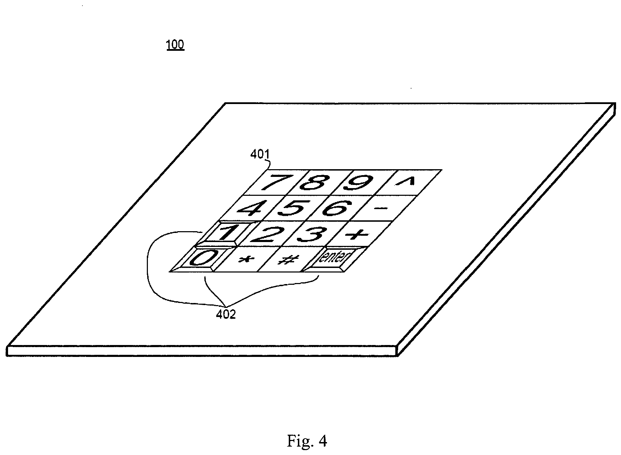

Figur 4 zeigt den Touchscreen mit taktilem Feedback 100 mit einem dargestellten Eingabefeld 401. Das Eingabefeld 401 ist beispielhaft als 10er Tastatur mit einigen Funktionstasten ausgeführt, dem Fachmann ist aber klar, dass jede beliebige Darstellung ebenso möglich ist.

Die Bedienfelder 402 ("0", "1" und "enter") sind in Figur 4 erhöht dargestellt. Dies kann beispielsweise eine erlaubte Eingabe kennzeichnen, die fühlbar ist. Ebenso können in schon beschriebener Weise Bedienfelder durch eine fühlbare Absenkung gegenüber den Umgebungsflächen, einer Vibration oder einer Änderung der gefühlten Rauhigkeit gekennzeichnet sein.

Durch die besonderen taktilen Eigenschaften von Teilflächen des Touchscreens, in Figur 4 als Erhöhung der Bedienfelder mit den zugeordneten Ziffern "0", "1" und "enter" dargestellt, erhöht sich der Eingabekomfort für den Bediener einerseits, und darüber hinaus erhöht sich auch die Sicherheit der Bedienung, da nicht plausible Eingaben durch das weitere Merkmal der taktilen Eigenschaft gekennzeichnet sind.

Mit Hilfe der Erfindung gelingt es, medizintechnische Geräte, die mit einem Touchscreen ausgerüstet sind, sicherer und komfortabel zu gestalten. Die vorliegende Erfindung ist nicht auf die vorstehend beschriebenen Ausführungsformen beschränkt, diese dienen lediglich der Veranschaulichung. Dem Fachmann liegt es nahe, die Merkmale der Erfindung aufzugreifen, um weitere Ausführungsformen auszugestalten.

Figur 4 zeigt den Touchscreen mit taktilem Feedback 100 mit einem dargestellten Eingabefeld 401. Das Eingabefeld 401 ist beispielhaft als 10er Tastatur mit einigen Funktionstasten ausgeführt, dem Fachmann ist aber klar, dass jede beliebige Darstellung ebenso möglich ist.

Die Bedienfelder 402 ("0", "1" und "enter") sind in Figur 4 erhöht dargestellt. Dies kann beispielsweise eine erlaubte Eingabe kennzeichnen, die fühlbar ist. Ebenso können in schon beschriebener Weise Bedienfelder durch eine fühlbare Absenkung gegenüber den Umgebungsflächen, einer Vibration oder einer Änderung der gefühlten Rauhigkeit gekennzeichnet sein.

Durch die besonderen taktilen Eigenschaften von Teilflächen des Touchscreens, in Figur 4 als Erhöhung der Bedienfelder mit den zugeordneten Ziffern "0", "1" und "enter" dargestellt, erhöht sich der Eingabekomfort für den Bediener einerseits, und darüber hinaus erhöht sich auch die Sicherheit der Bedienung, da nicht plausible Eingaben durch das weitere Merkmal der taktilen Eigenschaft gekennzeichnet sind.

Mit Hilfe der Erfindung gelingt es, medizintechnische Geräte, die mit einem Touchscreen ausgerüstet sind, sicherer und komfortabel zu gestalten. Die vorliegende Erfindung ist nicht auf die vorstehend beschriebenen Ausführungsformen beschränkt, diese dienen lediglich der Veranschaulichung. Dem Fachmann liegt es nahe, die Merkmale der Erfindung aufzugreifen, um weitere Ausführungsformen auszugestalten.

## Patentansprüche

1. Medizintechnisches Gerät mit mindestens einem Touchscreen, **dadurch gekennzeichnet, dass** der Touchscreen über zwei redundante Sensoren zur Detektion der Position eines Fingerdrucks auf den Touchscreen verfügt, wobei zumindest einer der Sensoren eine Vielzahl von Piezoelementen umfasst,
**dadurch gekennzeichnet, dass** das medizinische Gerät dazu eingerichtet ist, zu erkennen, ob die optische Anzeige des Touchscreens funktioniert und im Falle, dass erkannt wird, dass die optische Anzeige des Touchscreens nicht funktioniert, die Piezoelemente derart ansteuert, dass Teilflächen des Touchscreens unterschiedliche taktile Eigenschaften haben.

2. Medizintechnisches Gerät nach Anspruch 1, wonach das medizintechnische Gerät ein Blutbehandlungsgerät ist und insbesondere zur Hämodialyse, zur Hämofiltration, zur Hämodiafiltration, zur Plasmapherese oder zur automatischen Peritonealdialyse eingerichtet ist.

3. Medizinisches Gerät nach Anspruch 1, wonach das medizinische Gerät dazu eingerichtet ist, bei einem Fingerdruck auf einer der Teilflächen des Touchscreens eine vordefinierte Aktion des medizintechnischen Geräts auszulösen.

4. Medizintechnisches Gerät nach Anspruch 1, wonach die Piezoelemente dazu angesteuert werden können, sich zu verformen.

5. Medizintechnisches Gerät nach Anspruch 4, wonach die Verformung eine Vertiefung oder eine Erhöhung und/oder eine Vibration senkrecht zur Oberfläche des Touchscreens ist.

6. Medizintechnisches Gerät nach einem der vorhergehenden Ansprüche, wonach das medizintechnische Gerät ein Steuergerät beinhaltet, das dazu eingerichtet ist, jedes Piezoelement mit einer elektrischen Spannung zu belegen und/oder die elektrische Spannung, welche über jedem Piezoelement abfällt zu messen.

7. Medizintechnisches Gerät nach Anspruch 6, wonach das Steuergerät dazu eingerichtet ist aus den gemessenen Spannungen jedes Piezoelements die Positionen und die Krafteinwirkung jedes Fingerdrucks auf den Touchscreen zu ermitteln.

8. Verfahren zur Anzeige und Eingabe von Informationen an einem medizintechnischem Gerät mit mindestens einem Touchscreen, **dadurch gekennzeichnet, dass** zur Bestimmung der Position eines Fingerdrucks auf dem Touchscreen zwei redundante Sensoren benutzt werden, wobei zumindest ein Sensor zur Detektion der Position eines Fingerdrucks auf dem Touchscreen eine Vielzahl von Piezoelementen umfasst,
**dadurch gekennzeichnet, dass** erkannt werden kann, ob die optische Anzeige des Touchscreens funktioniert und im Falle, dass erkannt wird, dass die optische Anzeige des Touchscreens nicht funktioniert, die Piezoelemente derart angesteuert werden können, dass Teilflächen des Touchscreens unterschiedliche taktile Eigenschaften haben.

9. Verfahren nach Anspruch 8, wonach eine Steuervorrichtung vorhanden ist, die dazu eingerichtet ist, die Signale der zwei redundanten Sensoren zur Detektion der Position eines Fingerdrucks auf den Touchscreen zu erfassen und wonach die Steuervorrichtung auf einen Fehlerfall schließt, wenn nur ein Sensorsignal erfasst wird oder wonach die Steuervorrichtung auf einen unbeabsichtigten Fingerdruck schließt, wenn das Sensorsignal zu klein ist.

10. Verfahren nach dem Anspruch 8, wonach ein Fingerdruck auf einer der Teilflächen des Touchscreens eine vordefinierte Aktion des medizintechnischen Geräts auslöst.

11. Verfahren nach Anspruch 8, wonach die Steuervorrichtung dazu eingerichtet ist, individuelle Signale einzelner Piezoelemente aus der Vielzahl von Piezoelementen zur Detektion der Position eines Fingerdrucks auf den Touchscreen so auszuwerten, dass die Positionen mehrerer Finger, die gleichzeitig auf den Touchscreen drücken, ermittelt werden können.

## Claims

1. A medical device having at least one touchscreen, **characterized in that** the touchscreen has two redundant sensors for detecting the position of a fingerprint on the touchscreen, at least one of the sensors comprising a plurality of piezo elements
**characterized in that** the medical device is equipped to detect whether the optical display of the touchscreen is functioning, and in the event that it detects that the optical display of the touchscreen is not functioning, it controls the piezo elements in such a way that subareas of the touchscreen have different tactile properties.

2. The medical device according to claim 1, wherein the medical device is a blood treatment device, which is equipped in particular for hemodialysis, hemofiltration, hemodiafiltration, plasmapheresis or automatic peritoneal dialysis.

3. The medical device according to claim 1, wherein the medical device is equipped to trigger a predefined action of the medical device when a fingerprint is detected on a subarea of the touchscreen.

4. The medical device according to claim 1, wherein the piezo elements may be controlled to undergo deformation.

5. The medical device according to claim 4, wherein the deformation is an indentation or an elevation and/or a vibration orthogonal to the surface of the touchscreen.

6. The medical device according to any one of the preceding claims, wherein the medical device comprises a control unit, which is equipped to apply an electrical voltage to each piezo element and/or to measure the electrical voltage dropping across each piezo element.

7. The medical device according to claim 6, wherein the control unit is equipped to determine the positions and the force effect of each fingerprint on the touchscreen from the measured voltages of each piezo element.

8. A method for display and input of information on a medical device having at least one touchscreen, **characterized in that** two redundant sensors are used for a determination of the position of a fingerprint on the touchscreen, wherein at least one sensor comprises a plurality of piezo elements for detecting the position of a fingerprint on the touchscreen,
**characterized in that** it is possible to detect whether the optical display of the touchscreen is functioning, and in the event it detects that the optical display of the touchscreen is not functioning, the piezo elements can be controlled in such a way that subareas of the touchscreen have different tactile properties.

9. The method according to claim 8, wherein a control device is provided and is equipped to detect the signals of the two redundant sensors for detecting the position of a fingerprint on the touchscreen, and wherein the control device ascertains that there is a fault incident when only one sensor signal is detected, or wherein the control device concludes that there has been an unintentional fingerprint when the sensor signal too low.

10. The method according to claim 8, wherein a fingerprint on a subarea of the touchscreen triggers a predefined action by the medical device.

11. The method according to claim 8, wherein the control device is equipped to evaluate individual signals of individual piezo elements from the plurality of piezo elements for detecting the position of a fingerprint on the touchscreen, so that the positions of several fingers pressing on the touchscreen at the same time can be ascertained.

## Revendications

1. Appareil médical comportant un écran tactile, **caractérisé en ce que** l'écran tactile dispose de deux capteurs redondants pour la détection de la position d'une pression de doigt sur l'écran tactile, au moins un des capteurs comprenant une multitude d'éléments piézo-électriques,
**caractérisé en ce que** l'appareil médical est conçu pour détecter si l'affichage de l'écran tactile fonctionne et, dans le cas où il est détecté que l'affichage optique de l'écran tactile ne fonctionne pas, les éléments piézo-électriques sont commandés de manière à ce que des sous-surfaces de l'écran tactile aient des propriétés tactiles différents.

2. Appareil médical selon la revendication 1, l'appareil médical étant un appareil de traitement sanguin et étant conçu en particulier pour l'hémodialyse, l'hémofiltration, l'hémodiafiltration, la plasmaphérèse ou pour la dialyse péritonéale automatique.

3. Appareil médical selon la revendication 1, l'appareil médical étant conçu pour, en cas de pression du doigt sur une des sous-surfaces de l'écran tactile, déclencher une action prédéfinie de l'appareil médical.

4. Appareil médical selon la revendication 1, les éléments piézo-électriques pouvant être commandés pour se déformer.

5. Appareil médical selon la revendication 4, la déformation étant un creux ou une proéminence et/ou une vibration perpendiculaire à la surface supérieure de l'écran tactile.

6. Appareil médical selon une des revendications précédentes, l'appareil médical contenant un appareil de commande qui est conçu pour fournir à chaque élément piézo-électrique une tension électrique et/ou pour mesurer la tension électrique qui chute au-dessus de chaque élément piézo-électrique.

7. Appareil médical selon la revendication 6, l'appareil de commande étant conçu pour déterminer, à partir des tensions mesurées de chaque élément piézo-électrique, les positions et la force exercée par chaque pression de doigt sur l'écran tactile.

8. Procédé d'affichage et de saisie d'informations dans un appareil médical comportant au moins un écran tactile, **caractérisé en ce que**, pour déterminer la position d'une pression de doigt sur l'écran tactile, on utilise deux capteurs redondants, au moins un capteur de détection de la position d'une pression de doigt sur l'écran tactile comprenant une multitude d'éléments piézo-électriques,
**caractérisé en ce qu'**on peut détecter si l'affichage de l'écran tactile fonctionne et, dans le cas où il est détecté que l'affichage optique de l'écran tactile ne fonctionne pas, les éléments piézo-électriques peuvent être commandés de manière à ce que des sous-surfaces de l'écran tactile aient des propriétés tactiles différentes.

9. Procédé selon la revendication 8, étant prévu un dispositif de commande qui est conçu pour enregistrer des signaux des deux capteurs redondants pour la détection de la position d'une pression de doigt sur l'écran tactile et le dispositif de commande concluant à la présence d'une erreur si un seul signal de capteur est détecté ou le dispositif de commande concluant à une pression de doigt non intentionnelle si le signal de capteur est trop faible.

10. Procédé selon la revendication 8, une pression du doigt sur une des sous-surfaces de l'écran tactile déclenchant une action prédéfinie de l'appareil médical

11. Procédé selon la revendication 8, le dispositif de commande étant conçu pour exploiter des signaux individuels d'éléments piézo-électriques distincts parmi la multitude d'éléments piézo-électriques pour la détection de la position d'une pression de doigt sur l'écran tactile de manière à ce que les positions de plusieurs doigts qui appuient en même temps sur l'écran tactile puissent être déterminées.
